# EUROPEAN PATENT APPLICATION

(11) **EP 4 726 737 A1**
(43) Date of publication of application: **15.04.2026**
(21) Application number: 24819494.6
(22) Date of filing: 09.05.2024
(51) Int. Cl.: G16H 50/50, G16H 30/00, G16H 50/70, G06T 7/00, G06N 3/08, G06V 10/46, G06V 10/25

(54) **METHOD AND DEVICE FOR ANALYZING PATHOLOGY SLIDE IMAGE**

(30) Priority: 09.06.2023 KR 20230074471
(71) Applicant: Lunit Inc., Seoul 06241 (KR)
(72) Inventor: LEE, Tae Bum, Seoul 06241 (KR); CHO, Soo Ick, Seoul 06241 (KR); JUNG, Won Kyung, Seoul 06241 (KR); SONG, Sang Hoon, Seoul 06241 (KR)
(74) Representative: AWA Sweden AB
(86) International application number: PCT/KR2024/006298
(87) International publication number: WO 2024/253342

(57) **Abstract**

A computing apparatus according to an aspect includes: at least one memory; and at least one processor, wherein the at least one processor is configured to identify at least one tertiary lymphoid structure (TLS) from a pathological slide image, generate information related to the at least one tertiary lymphoid structure, and predict treatment responsiveness of a lesion associated with the pathological slide image, on the basis of the information.

## Description

### Technical Field

The present disclosure relates to a method and apparatus for analyzing a pathological slide image.

### Background Art

The field of digital pathology refers to a field that acquires histological information regarding corresponding patients or predicts prognosis of the patients by using whole slide images generated by scanning pathological slide images.

Recently, technologies for predicting medical information by analyzing pathological slide images through artificial intelligence models have been developed. However, there is a need for a method of analyzing a pathological slide image, which is for increasing accuracy and speed when predicting medical information.

### Disclosure of Invention

### Technical Problem

Provided are a method and apparatus for analyzing a pathological slide image. In addition, provided is a computer-readable recording medium having recorded thereon a program for causing a computer to execute the method. The technical problems to be solved are not limited to the technical problems as described above, and other technical problems may be present.

### Solution to Problem

A computing apparatus according to an aspect includes: at least one memory; and at least one processor, wherein the at least one processor is configured to identify at least one tertiary lymphoid structure (TLS) from a pathological slide image, generate information related to the at least one tertiary lymphoid structure, and predict treatment responsiveness of a lesion associated with the pathological slide image, on the basis of the information.

A method of analyzing a pathological slide image, according to another aspect, includes: identifying at least one tertiary lymphoid structure from a pathological slide image; generating information related to the at least one tertiary lymphoid structure; and predicting treatment responsiveness of a lesion associated with the pathological slide image, on the basis of the information.

A computer-readable recording medium according to another aspect includes a recording medium having recorded thereon a program for causing a computer to execute the method described above.

### Advantageous Effects of Invention

According to the problem solving means of the present disclosure described above, a method of predicting medical information at a high speed and with high accuracy may be provided.

In addition, according to the problem solving means of the present disclosure described above, a prognosis for immune treatment may be predicted by using a new biomarker related to a tertiary lymphoid structure.

### Brief Description of Drawings

FIG. 1 is a view illustrating an example of a system for analyzing a pathological slide image, according to an embodiment.
FIG. 2A is a block diagram illustrating an example of a user terminal according to an embodiment.
FIG. 2B is a block diagram illustrating an example of a server according to an embodiment.
FIG. 3 is a flowchart illustrating an example of a method of analyzing a pathological slide image, according to an embodiment.
FIG. 4 is a view illustrating an example of acquiring a pathological slide image, according to an embodiment.
FIG. 5 is a view illustrating an example of a pathological slide image according to an embodiment.
FIG. 6 is a view illustrating a method of calculating a quantified value on the basis of a parameter, according to an embodiment of the present disclosure.
FIGS. 7A to 7C are views illustrating classification according to a location of a tertiary lymphoid structure 710, according to an embodiment of the present disclosure.
FIGS. 8A to 8C are views illustrating classification according to maturation of a tertiary lymphoid structure, according to an embodiment of the present disclosure.
FIG. 9 is a view illustrating a region of interest according to an embodiment of the present disclosure.
FIG. 10 is a view illustrating an example of an image displaying TLS information, according to an embodiment of the present disclosure.
FIG. 11 is a view illustrating another example of a system for analyzing a pathological slide image.

### Best Mode for Carrying out the Invention

A computing apparatus according to an aspect includes: at least one memory; and at least one processor, wherein the at least one processor is configured to identify at least one tertiary lymphoid structure (TLS) from a pathological slide image, generate information related to the at least one tertiary lymphoid structure, and predict treatment responsiveness of a lesion associated with the pathological slide image, on the basis of the information.

### Mode for the Invention

Terms used in embodiments are selected as currently widely used general terms as possible, which may vary depending on intentions or precedents of those skilled in the art, emergence of new technologies, and the like. In addition, in certain cases, there are also terms arbitrarily selected by the applicant, and in this case, the meaning thereof will be defined in detail in the description. Therefore, the terms used herein should be defined based on the meanings of the terms and the details throughout the description, rather than the simple names of the terms.

Throughout the description, when a part includes a certain element, it means that other elements may be further included, rather than excluding the other elements, unless otherwise stated. In addition, the term, such as "~ unit" or "~ module" described herein, refers to a unit that processes at least one function or operation, which may be implemented as hardware or software, or a combination of hardware and software.

Also, although the terms, "first," "second," etc. may be used herein to describe various components, the components should not be limited by the terms. The terms may be used to distinguish one component from another component.

According to an embodiment, "a pathological slide image" may refer to an image obtained by capturing a pathological slide that is fixed and stained via a series of chemical treatment processes for a tissue or the like removed from a human body. In addition, the pathological slide image may refer to a whole slide image (WSI) including a high-resolution image of a whole slide, and may also refer to a portion of the whole slide image, for example, one or more patches. For example, the pathological slide image may refer to a digital image captured or scanned via a scanning apparatus (e.g., a digital scanner or the like), and may include information regarding a particular protein, cell, tissue and/or structures within a human body. In addition, the pathological slide image may include one or more patches, and histological information may be applied (e.g., tagged) to the one or more patches via an annotation task.

"Medical information" may refer to any medically meaningful information that may be extracted from a medical image. For example, the medical information may include at least one of an immune phenotype, a genotype, a biomarker score, tumor purity, information regarding RNA, information regarding a tumor microenvironment, and a treatment method for cancer expressed in a pathological slide image.

Also, the medical information may include an area, location, and size of a particular tissue (e.g., a cancer tissue, a cancer stromal tissue, or the like) and/or a particular cell (e.g., a tumor cell, a lymphocyte cell, a macrophage cell, an endothelial cell, a fibroblast cell, or the like) within a medical image, diagnostic information regarding cancer, information associated with the possibility of developing cancer of a subject, and/or a medical conclusion associated with cancer treatment, but is not limited thereto.

In addition, the medical information may include not only a quantified numerical value that may be obtained from a medical image, but also information obtained by visualizing the numerical value, predictive information according to the numerical value, image information, statistical information, and the like.

For example, the medical information may be provided to a user terminal or output through a display apparatus.

Hereinafter, embodiments are described in detail with reference to the accompanying drawings. However, the embodiments may be implemented in several different forms and are not limited to examples described herein.

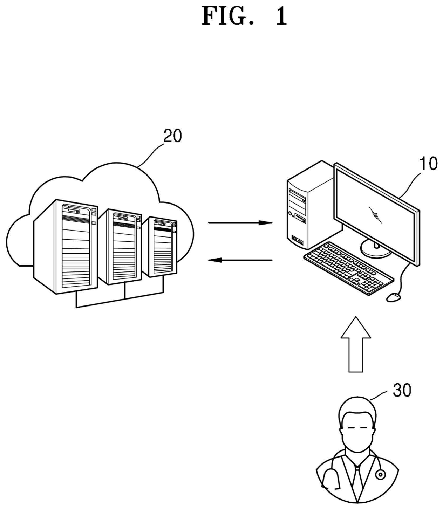

FIG. 1 is a view illustrating an example of a system for analyzing a pathological slide image, according to an embodiment.

Referring to FIG. 1, a system for analyzing a pathological slide image may include a user terminal 10 and a server 20. For example, the user terminal 10 and the server 20 may be connected to each other by a wired or wireless communication method to transmit and/or receive various types of data to and/or from each other.

For convenience of description, although FIG. 1 illustrates that the system includes the user terminal 10 and the server 20, the present disclosure is not limited thereto. For example, the system may include another external device (not shown). In addition, operations of user terminal 10 and server 20 described below may be implemented by a single device (e.g., the user terminal 10 or the server 20) or more devices.

The user terminal 10 may be a computing apparatus that is provided with a display apparatus and a device (e.g., a keyboard, a mouse, or the like) for receiving a user input, and includes a memory and a processor. Also, the display apparatus may be implemented as a touch screen to perform a function of receiving a user input. For example, the user terminal 10 may correspond to a notebook PC, a desktop PC, a laptop, a tablet computer, a smartphone, or the like, but is not limited thereto.

The server 20 may be an apparatus that communicates with an external device (not shown) including the user terminal 10. For example, the server 20 may be an apparatus that stores various types of data including a pathological slide image, a bitmap image corresponding to the pathological slide image, information generated by analysis of the pathological slide image (e.g., including information regarding an area of at least one cell, tissue, or structure expressed in the pathological slide image, information regarding at least one biomarker expression, medical information related to the pathological slide image, and the like), and information regarding an artificial intelligence model used for analysis of the pathological slide image. Alternatively, the server 20 may be a computing apparatus including a memory and a processor, and having an arithmetic operation capability. In the case where the server 20 is a computing apparatus, the server 20 may perform at least some of operations of the user terminal 10 described below with reference to FIGS. 1 to 11. For example, the server 20 may also be a cloud server, but is not limited thereto.

The user terminal 10 outputs the pathological slide image and/or information generated via analysis of the pathological slide image. For example, the user terminal 10 may output various types of information regarding an area of at least one cell, tissue, or structure expressed in the pathological slide image. In addition, the user terminal 10 may output expression information of a biomarker.

The pathological slide image may refer to an image obtained by capturing a pathological slide that is fixed and stained through a series of chemical treatment processes to observe a tissue or the like removed from a human body under a microscope. As an example, the pathological slide image may refer to a whole slide image including a high-resolution image for a whole slide. As another example, the pathological slide image may refer to a portion of the high-resolution whole slide image.

Meanwhile, the pathological slide image may refer to an area segmented into patch (or tile) units from the whole slide image. For example, a patch (or a tile) may have a size of a certain area.

In addition, the pathological slide image may refer to a digital image captured by using a microscope and may include information regarding a cell, a tissue, and/or structures within a human body.

Biological factors (e.g., a cancer cell, an immune cell, a tumor area, a stroma area, a particular structure, and the like) expressed in the pathological slide image may be identified via analysis of the pathological slide image. The biological factors may be used for a histological diagnosis of a disease, prediction of a disease prognosis, a determination of a treatment direction for a disease, or the like.

The user terminal 10 may analyze the pathological slide image by using an artificial intelligence model. For example, the user terminal 10 may individually detect many cells distributed on the pathological slide image and segment each area from a tissue, by using the artificial intelligence model. In addition, the user terminal 10 may analyze the detected cells and the segmented areas and derive the result of the analysis, by using the artificial intelligence model.

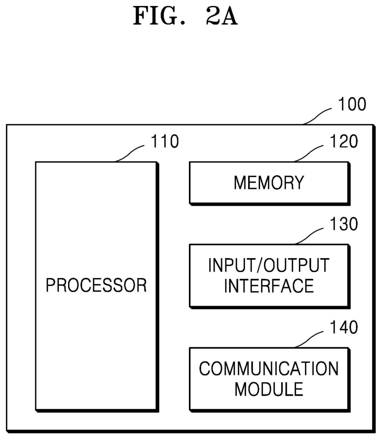

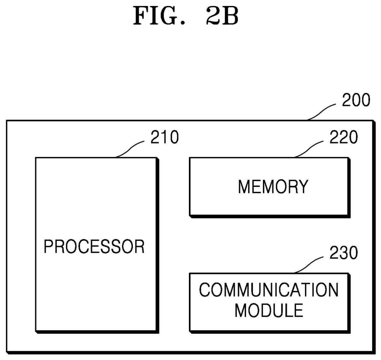

Hereinafter, an example in which the user terminal 10 analyzes a pathological slide image is described with reference to FIGS. 2 to 11.

Meanwhile, for convenience of description, the user terminal 10 has been described as performing all operations throughout the description, but is not limited thereto. For example, at least some of the operations performed by the user terminal 10 may also be performed by the server 20.

FIG. 2A is a block diagram illustrating an example of a user terminal according to an embodiment.

Referring to FIG. 2A, a user terminal 100 includes a processor 110, a memory 120, an input/output interface 130, and a communication module 140. For convenience of description, FIG. 2A illustrates only components related to the present disclosure. Accordingly, the user terminal 100 may further include other general-purpose components in addition to the components illustrated in FIG. 2A. In addition, it is obvious to those skilled in the art related to the present disclosure that the processor 110, the memory 120, the input/output interface 130, and the communication module 140 illustrated in FIG. 2A may be implemented as independent devices.

The processor 110 may process commands of a computer program by performing basic arithmetic, logic, and input/output operations. Here, the commands may be provided from the memory 120 or an external apparatus (e.g., the server 200 or the like). In addition, the processor 110 may generally control operations of other components included in the user terminal 100.

The processor 110 may identify a tertiary lymphoid structure from a pathological slide image. In addition, the processor 110 may identify a component including at least one of a tumor area, a stroma area, and an immune cell from the pathological slide image.

The processor 110 may identify at least one tertiary lymphoid structure by using an artificial intelligence model trained on the basis of a plurality of training images. Here, the plurality of training images may be obtained by labeling the tertiary lymphoid structure on the pathological slide image.

The processor 110 may generate information related to the identified tertiary lymphoid structure. As an example, the processor 110 may calculate a quantified value on the basis of at least one of a first parameter related to the tertiary lymphoid structure and a second parameter related to a component other than the tertiary lymphoid structure. In detail, the first parameter may include at least one of a location of the tertiary lymphoid structure, an area of the tertiary lymphoid structure, and maturation of the tertiary lymphoid structure, and the second parameter may include at least one of a location of a tumor area, an area of the tumor area, a location of a stroma area, an area of the stroma area, a location of an immune cell, and the number of immune cells.

Meanwhile, the quantified value may be a value reflecting structural information associated with treatment responsiveness of the tertiary lymphoid structure within the pathological slide image. As an example, the quantified value may include at least one of the number of tertiary lymphoid structures per unit area of a tumor area, a ratio of an area of the tumor area to an area of a tertiary lymphoid structure, the number of tertiary lymphoid structures within the tumor area, the number of tertiary lymphoid structures around the tumor area, a distance between the tumor area and a tertiary lymphoid structure, a distance between a plurality of tertiary lymphoid structures, a size of a tertiary lymphoid structure, the number of tertiary lymphoid structures forming a germinal center, the number of tumor infiltrating lymphocytes located within a certain radius from a tertiary lymphoid structure, and a density of an immune cell within a tertiary lymphoid structure.

The processor 110 may generate information related to a tertiary lymphoid structure within a region of interest determined on the basis of a distance from a tumor area.

The processor 110 may predict treatment responsiveness of a lesion associated with the pathological slide image on the basis of the information related to the tertiary lymphoid structure. For example, the processor 110 may classify a class of the pathological slide image on the basis of a preset cut-off value for the information.

Meanwhile, the processor 110 may output a plurality of detection targets (e.g., a component including at least one of a tumor area, a stroma area, and an immune cell) including the tertiary lymphoid structure identified from the pathological slide image. In addition, the processor 110 may output a report including the information related to the tertiary lymphoid structure. In addition, the processor 110 may output a report including the treatment responsiveness of the lesion associated with the pathological slide image. For example, processor 110 may control a display apparatus to output at least one of the information related to the tertiary lymphoid structure described above, the treatment responsiveness predicted on the basis of the information related to the tertiary lymphoid structure, and the report.

The processor 110 may be implemented as an array of a plurality of logical gates, or may be implemented as a combination of a general-purpose microprocessor and a memory that stores a program executable by the microprocessor. For example, the processor 110 may include a general-purpose processor, a central processing unit (CPU), a microprocessor, a digital signal processor (DSP), a controller, a microcontroller, a state machine, or the like. In some environments, the processor 110 may include an application-specific integrated circuit (ASIC), a programmable logic device (PLD), a field programmable gate array (FPGA), or the like. For example, the processor 110 may refer to a combination of processing devices, such as a combination of a digital signal processor (DSP) and a microprocessor, a combination of a plurality of microprocessors, a combination of one or more microprocessors combined with a digital signal processor (DSP) core, or any other combination of such components.

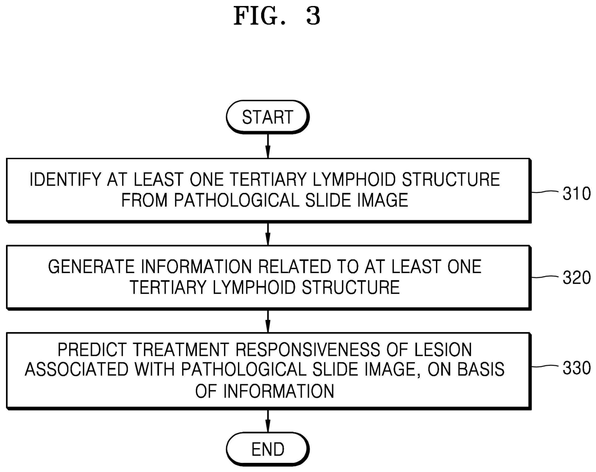

The memory 120 may include any non-transitory computer-readable recording medium. As an example, the memory 120 may include a permanent mass storage device such as random access memory (RAM), read only memory (ROM), a disk drive, a solid state drive (SSD), or flash memory. As another example, the permanent mass storage device such as ROM, an SSD, flash memory, or a disk drive may be a separate permanent storage device that is distinguished from a memory. In addition, the memory 120 may store an operating system (OS) and at least one program code (e.g., a code for the processor 110 to perform an operation described below with reference to FIGS. 3 to 11).

Software components described above may be loaded from a computer-readable recording medium that is separate from the memory 120. The separate computer-readable recording medium may be a recording medium that may be directly connected to the user terminal 100 and may include, for example, a computer-readable recording medium such as a floppy drive, a disk, a tape, a DVD/CD-ROM drive, or a memory card. Alternatively, the software components may also be loaded into the memory 120 through the communication module 140 rather than the computer-readable recording medium. For example, the at least one program may be loaded into the memory 120 on the basis of a computer program that is installed by files provided through the communication module 140 by developers or a file distribution system that distributes installation files of applications (e.g., a computer program or the like for the processor 110 to perform operations described below with reference to FIGS. 3 to 11).

The input/output interface 130 may be a unit for interfacing with a device (e.g., a keyboard, a mouse, or the like) for an input or output, which may be connected to the user terminal 100 or may be included in the user terminal 100. Although FIG. 2A illustrates the input/output interface 130 as a component configured separately from the processor 110, but the input/output interface 130 is not limited thereto and may also be configured to be included in the processor 110.

The communication module 140 may provide a component or function for the server 200 and the user terminal 100 to communicate with each other through a network. In addition, the communication module 140 may provide a component or function for the user terminal 100 to communicate with another external device. For example, a control signal, a command, data, and the like, which are provided under control of the processor 110, may be transmitted to the server 200 and/or an external device through the communication module 140 and the network.

Meanwhile, although not illustrated in FIG. 2A, the user terminal 100 may further include a display apparatus. Alternatively, the user terminal 100 may be connected to an independent display apparatus through a wired or wireless communication method to transmit and receive data to and from each other. For example, a pathological slide image, analysis information regarding the pathological slide image, medical information, additional information based on the medical information, and the like may be provided to a user 30 through the display apparatus.

FIG. 2B is a block diagram illustrating an example of a server according to an embodiment.

Referring to FIG. 2B, a server 200 includes a processor 210, a memory 220, and a communication module 230. For convenience of description, FIG. 2B illustrates only components related to the present disclosure. Accordingly, the server 200 may further include other general-purpose components in addition to the components illustrated in FIG. 2B. In addition, it is obvious to those skilled in the art related to the present disclosure that the processor 210, the memory 220, and the communication module 230 illustrated in FIG. 2B may be implemented as independent devices.

The processor 210 may acquire a pathological slide image from at least one of the internal memory 220, the user terminal 100, and another external device. The processor 210 may identify at least one tertiary lymphoid structure from the pathological slide image, generate information related to the at least one tertiary lymphoid structure, predict treatment responsiveness of a lesion associated with the pathological slide image, on the basis of the generated information, or transmit at least one of the identified at least one tertiary lymphoid structure, the generated information, and the predicted treatment responsiveness to the user terminal 100. In addition, the processor 210 may transmit a report (e.g., including a classified class of the pathological slide image or the treatment responsiveness) to the user terminal 100.

In other words, at least one of the operations of the processor 110 described above with reference to FIG. 2A may be performed by the processor 210. In this case, the user terminal 100 may output, through a display apparatus, information transmitted from the server 200.

Meanwhile, an implementation example of the processor 210 is the same as the implementation example of the processor 110 described above with reference to FIG. 2A, and thus, a detailed description thereof is omitted.

The memory 220 may store various types of data such as the pathological slide image and data generated according to the operation of the processor 210. In addition, the memory 220 may store an operating system (OS) and at least one program (e.g., a program needed for the processor 210 to operate, and the like).

Meanwhile, an implementation example of the memory 220 is the same as the implementation example of the memory 120 described above with reference to FIG. 2A, and thus, a detailed description thereof is omitted.

The communication module 230 may provide a component or function for the server 200 and the user terminal 100 to communicate with each other through a network. In addition, the communication module 230 may provide a component or function for the server 200 to communicate with another external device. For example, a control signal, a command, data, and the like, which are provided under control of the processor 210, may be transmitted to the user terminal 100 and/or an external device through the communication module 230 and the network.

FIG. 3 is a flowchart illustrating an example of a method of analyzing a pathological slide image, according to an embodiment.

The method illustrated in FIG. 3 includes operations processed in a time series by the user terminal 10 or 100 or the processor 110 illustrated in FIG. 1 or 2A. Accordingly, although the above description of the user terminal 10 or 100 or the processor 110 illustrated in FIG. 1 or 2A is omitted below, the above description may also be applied to the method illustrated in FIG. 3.

In addition, as described above with reference to FIGS. 1 to 2B, at least one of the operations of the method illustrated in FIG. 3 may be processed by the server 20 or 200 or the processor 210.

In operation 310, a processor identifies at least one tertiary lymphoid structure tissue area from a pathological slide image.

In the present disclosure, the pathological slide image may be a whole slide image or a portion of the whole slide image. Here, the portion may be referred to as a patch or a tile.

In an embodiment, the processor may identify and classify a tumor area, a stroma area, a necrosis area, a background area, and the like from the pathological slide image by analyzing the pathological slide image. In addition, the processor may classify a plurality of cells expressed in the pathological slide image into at least one of a tumor cell, an immune cell including a lymphocyte cell, and other cells. Here, the lymphocyte cell may include a tumor infiltrating lymphocyte cell. The processor may determine locations and areas of the identified tumor area, stroma area, necrosis area, and background area, and/or a location and an area of a tertiary lymphoid structure. In addition, the processor may determine the number of the plurality of cells. In addition, the processor may determine structural and biological information of components identified from the pathological slide image as well as the tertiary lymphoid structure.

In addition, expression information of a biomarker may include the number of positive (+)/negative (-) tumor cells for programmed death-ligand 1 (PD-L1), the number of positive (+)/negative (-) immune cells, a tumor proportion score (TPS), a combined positive score (CPS), an immune phenotype, and the like. However, the expression information of the biomarker is not limited to the above description.

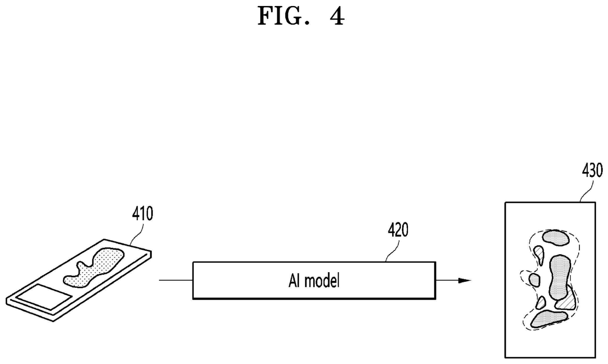

FIG. 4 is a view illustrating an example of acquiring a pathological slide image, according to an embodiment.

Referring to FIG. 4, in an embodiment, a processor may identify at least one tertiary lymphoid structure from a pathological slide image 430. In detail, the processor may identify the at least one tertiary lymphoid structure included in the pathological slide image 430 that is an image obtained by capturing a pathological slide 410. The pathological slide image 430 may be an image obtained by capturing the pathological slide 410 that is fixed and stained via a series of chemical treatment processes for a tissue or the like removed from a human body.

In an embodiment, the processor may identify the at least one tertiary lymphoid structure by using an artificial intelligence model 420. Here, the artificial intelligence model 420 may be a machine learning model. As an example, the artificial intelligence model 420 may be trained to infer histological information regarding all or a portion of the pathological slide image 430 (e.g., at least one patch included in the pathological slide image 430). In this case, histological information generated through an annotation task may be used to train the artificial intelligence model 420. For example, the histological information may include information (e.g., the number of particular cells and information regarding a tissue in which the particular cells are arranged) regarding a cell (e.g., a tumor cell, lymphocyte, macrophage, a dendritic cell, fibroblast, an endothelial cell, or the like) within a patch, but is not limited thereto. As another example, the artificial intelligence model 420 may be trained to infer characteristics of at least one of a cell, a tissue, and a structure within the pathological slide image 430.

In an embodiment, the artificial intelligence model 420 may identify a plurality of detection targets such as a tertiary lymphoid structure, a tumor area, a stroma area, and an immune cell by determining, by using the histological information, information regarding an area in which a cell is arranged in the pathological slide image 430.

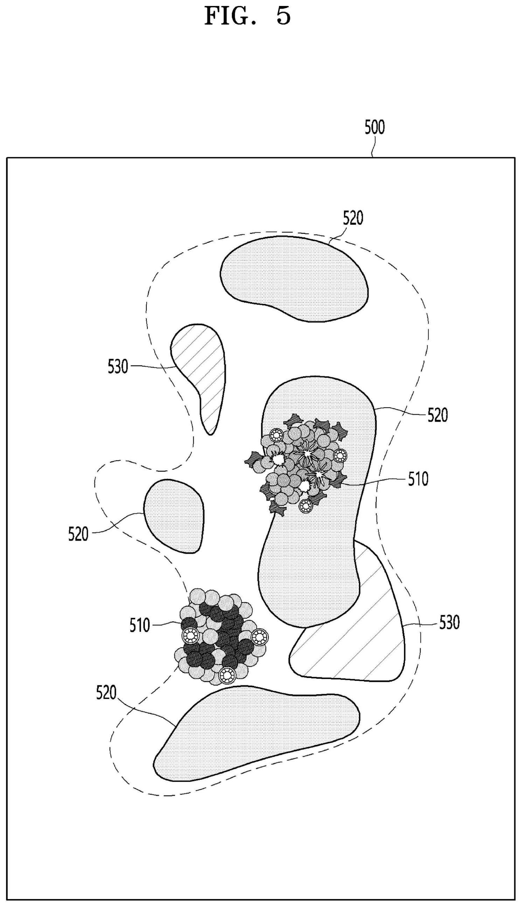

FIG. 5 is a view illustrating an example of a pathological slide image according to an embodiment.

FIG. 5 illustrates a plurality of detection targets included in a pathological slide image 500. For example, the detection targets may include a tertiary lymphoid structure 510 and components other than the tertiary lymphoid structure 510.

The tertiary lymphoid structure 510 is an organized aggregate of immune cells formed in a non-lymphoid tissue. The tertiary lymphoid structure 510 may affect a prognosis of immune treatment, in a relation with a tumor area 520 and thus needs to be researched and developed as a biomarker.

Meanwhile, components other than the tertiary lymphoid structure 510 may include at least one of a tissue area and an immune cell. A tissue is a collection of cells specialized to perform a common function, and a certain area in which the collection of cells is clustered is referred to as a tissue area. In an embodiment, the tissue area may include the tumor area 520 and a stroma area 530. The tumor area 520 refers to an area in which tumor cells showing an invasion phenomenon are clustered. The stroma area 530 refers to a peripheral area of the tumor area 520 in which tumor-related stroma changes, such as desmoplasia or aggregation of lymphoid cells, are observed.

The immune cell is a portion of an immune system and refers to a cell that detects and destroys an external pathogen or an abnormal cell in response to an infection, an allergy, a tumor, or the like. The immune cell may include a lymphocyte including a T cell and a B cell, a natural killer (NK) cell, a dendritic cell, a macrophage, neutrocyte, eosinophil, basophil, and the like. The tertiary lymphoid structure 510 is an organized aggregate of immune cells as described above.

In an embodiment, the histological information generated through the annotation task, which is described above with reference to FIG. 4, may be a plurality of training images in which all tertiary lymphoid structures included in an image are labeled on any pathological slide image. Accordingly, an artificial intelligence model may be a model trained on the basis of the plurality of training images. As an example, the artificial intelligence model may be an artificial neural network-based detection model. Accordingly, when the pathological slide image 500 is input, as is trained, the artificial intelligence model may identify the tertiary lymphoid structure 510.

Referring back to FIG. 3, in operation 320, the processor generates information related to the at least one tertiary lymphoid structure.

A tumor area in which a tertiary lymphoid structure is present internally or in proximity is related to an improved prognosis and clinical result of immune treatment. Therefore, a quantification method of using a tertiary lymphoid structure as a biomarker in relation to a tumor area, or in the tumor area is needed. Accordingly, the processor may generate information related to the tertiary lymphoid structure identified from the pathological slide image.

In an embodiment, the processor may calculate a quantified value on the basis of at least one of a first parameter related to the tertiary lymphoid structure and a second parameter related to a component other than the tertiary lymphoid structure.

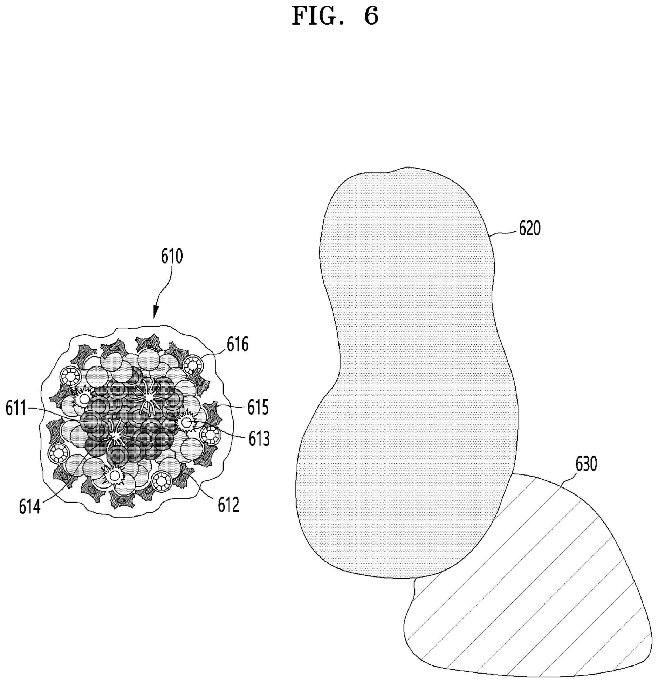

FIG. 6 is a view illustrating a method of calculating a quantified value on the basis of a parameter, according to an embodiment of the present disclosure.

FIG. 6 illustrates a tertiary lymphoid structure 610 including B cells 611, T cells 612, dendritic cells (DCs) 613, follicular dendritic cells (FDCs) 614, fibroblastic reticular cells (FRCs) 615, and high endothelial venules (HEVs) 616, a tumor area 620, and a stroma area 630.

Here, immune cells (the B cells 611, the T cells 612, the dendritic cells 613, the follicular dendritic cells 614, the fibroblastic reticular cells 615, and high endothelial venules 616) included in the tertiary lymphoid structure 610 are only examples, some of the examples may be omitted, and the tertiary lymphoid structure 610 may further include immune cells not illustrated in FIG. 6. In addition, the immune cells (611 to 616) may be located inside the tertiary lymphoid structure 610 to form the tertiary lymphoid structure 610, but may also be located outside the tertiary lymphoid structure 610.

Not only structural characteristics and biological characteristics of the tertiary lymphoid structure 610, but also areas of the tumor area 620 and the stroma area 630 or a relative location of the tertiary lymphoid structure 610 to the tumor area 620 and the stroma area 630 also affect immune treatment responsiveness to a tumor. Therefore, the processor may generate information related to the tertiary lymphoid structure 610 (hereinafter, referred to as "TLS information") by considering both a first parameter related to the tertiary lymphoid structure 610 and a second parameter related to an area including at least one of the tumor area 620, the stroma area 630, and the immune cells 611 to 616.

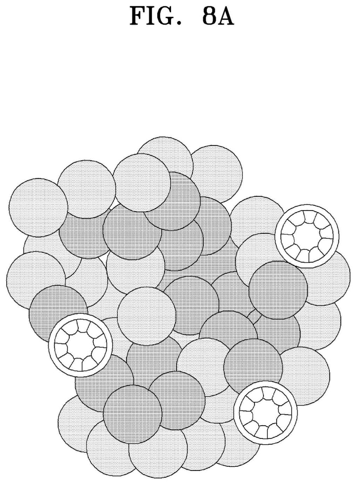

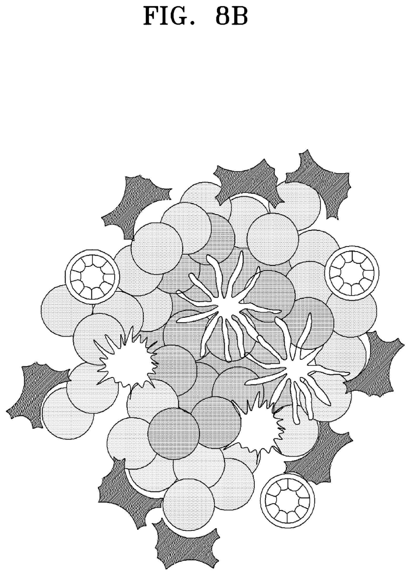

In an embodiment, the first parameter may include at least one of the number of tertiary lymphoid structures 610, a location of the tertiary lymphoid structure 610, an area of the tertiary lymphoid structure 610, and maturation of the tertiary lymphoid structure 610. For example, the location of the tertiary lymphoid structure 610 may be defined as (x, y) coordinate values within a pathological slide image, and the area of the tertiary lymphoid structure 610 may be expressed in a unit of mm². The maturation of the tertiary lymphoid structure 610 is described below with reference to FIGS. 8A to 8C.

In an embodiment, the second parameter may include at least one of the location of the tumor area 620, the area of the tumor area 620, the location of the stroma area 630, the area of the stroma area 630, locations of the immune cells 611 to 616, and the number of immune cells 611 to 616. The locations of the tumor area 620, the stroma area 630, and the immune cells 611 to 616 may be defined as (x, y) coordinate values within the pathological slide image, and the areas of the tumor area 620 and the stroma area 630 may be expressed in the unit of mm². The number of immune cells 611 to 616 may refer to the number using one cell as a unit.

In an embodiment, the processor may calculate the quantified value on the basis of at least one of the first parameter and the second parameter. In detail, the quantified value may be a value reflecting structural information associated with treatment responsiveness of the tertiary lymphoid structure 610 within the pathological slide image.

As an example, the TLS information may include at least one of the number of tertiary lymphoid structures 610 per unit area of the tumor area 620, a ratio of the area of the tumor area 620 to an area of the tertiary lymphoid structure 610, the number of tertiary lymphoid structures 610 inside the tumor area 620, the number of tertiary lymphoid structures 610 around the tumor area 620, a distance between the tumor area 620 and the tertiary lymphoid structure 610, a distance between a plurality of tertiary lymphoid structures 610, a size of the tertiary lymphoid structure 610, the number of tertiary lymphoid structures 610 forming a germinal center, the number of tumor infiltrating lymphocytes located within a certain radius from the tertiary lymphoid structure 610, and a density of the immune cells 611 to 616 within the tertiary lymphoid structure 610.

In detail, the number of tertiary lymphoid structures 610 per unit area of the tumor area 620 may be calculated by the processor from the number of tertiary lymphoid structures 610 that are the first parameter, and the area of the tumor area 620 that is the second parameter, and may be calculated in a unit of (number)/mm². In addition, the ratio of the area of the tumor area 620 to the area of the tertiary lymphoid structure 610 may be calculated by the processor from the area of the tertiary lymphoid structure 610 that is the first parameter and the area of the tumor area 620 that is the second parameter. The distance between the tumor area 620 and the tertiary lymphoid structure 610 may be calculated by the processor from the location of the tertiary lymphoid structure 610 that is the first parameter and the location of the tumor area 620 that is the second parameter.

The distance between the plurality of tertiary lymphoid structures 610 may be calculated by the processor from the location of the tertiary lymphoid structure 610 that is the first parameter. Also, the size of the tertiary lymphoid structure 610 may be generated by the processor by referring to the first parameter.

The number of tumor infiltrating lymphocytes located within a certain radius from the tertiary lymphoid structure 610 may be calculated by the processor from the location of the tertiary lymphoid structure 610 that is the first parameter and the locations of the immune cells 611 to 616 that are the second parameter. For example, being "located within the certain radius" may indicate being located in the stroma area 630, and the "certain radius" may be a radius between 2.5 mm and 5 mm and preset, but is not limited thereto. Meanwhile, the tumor infiltrating lymphocyte may include the B cells 611, the T cells 612, the natural killer (NK) cells (not shown), and the like.

The density of the immune cells 611 to 616 within the tertiary lymphoid structure 610 may be calculated by the processor from the location and area of the tertiary lymphoid structure 610 that are the first parameter, and the locations of the immune cells 611 to 616 and the number of immune cells 611 to 616 that are the second parameter.

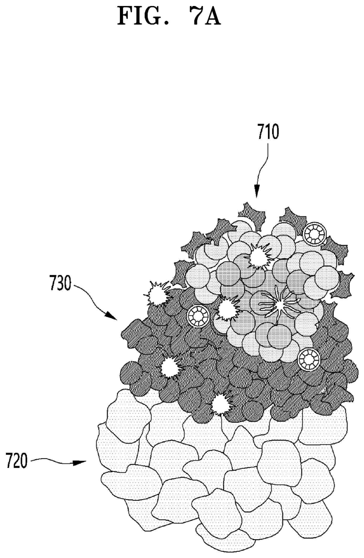

FIGS. 7A to 7C are views illustrating classification according to a location of a tertiary lymphoid structure 710, according to an embodiment of the present disclosure.

In an embodiment, a processor may generate, as TLS information, at least one of the number of tertiary lymphoid structures 710 inside a tumor area 720 and the number of tertiary lymphoid structures 710 around the tumor area 720. In detail, the processor may calculate the number of tertiary lymphoid structures 710 inside the tumor area 720 and the number of tertiary lymphoid structures 710 around the tumor area 720 from a location of the tumor area 720 (and/or a location of a stroma area) that is a second parameter and a location of the tertiary lymphoid structure 710 that is a first parameter.

In an embodiment, a tertiary lymphoid structure may be classified as stromal, peritumoral, and intratumoral according to a relative location thereof with a tumor area.

FIG. 7A illustrates a stromal tertiary lymphoid structure 710. The stromal tertiary lymphoid structure 710 refers to a structure in which all or a portion of the tertiary lymphoid structure 710 is not located inside a tumor area 720 but is located in a stroma area 730 that is in contact with the tumor area 720. As an example, all or a portion of the stromal tertiary lymphoid structure 710 may be located within a certain radius from the stroma area 630, and the certain radius may be preset to a radius between 2.5 mm and 5 mm, but is not limited thereto.

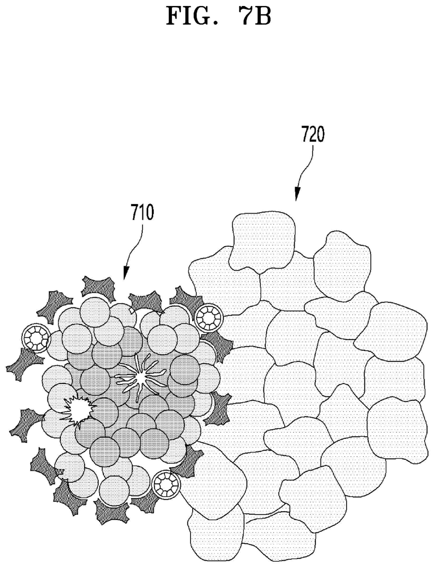

FIG. 7B illustrates a peritumoral tertiary lymphoid structure 710. The peritumoral tertiary lymphoid structure 710 refers to a structure in which all of the tertiary lymphoid structure 710 is not located inside a tumor area 720, but a portion thereof is located inside the tumor area 720.

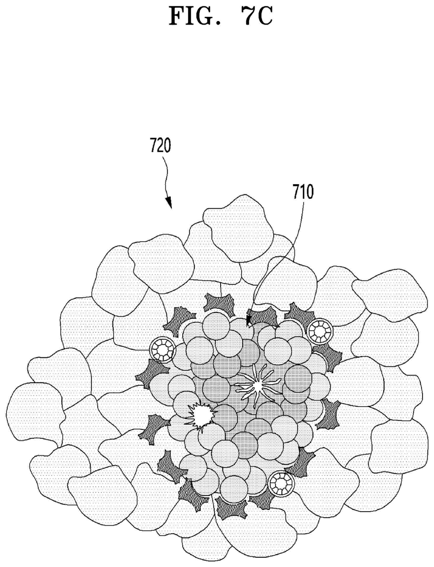

FIG. 7C illustrates an intratumoral tertiary lymphoid structure 710. The intratumoral tertiary lymphoid structure 710 refers to a structure in which all of the tertiary lymphoid structure 710 is located inside a tumor area 720.

As an example, the tertiary lymphoid structure 710 inside the tumor area 720 may include the intratumoral tertiary lymphoid structure 710 of FIG. 7C. The tertiary lymphoid structure 710 around the tumor area 720 may include at least one of the peritumoral tertiary lymphoid structure 710 of FIG. 7B and the stromal tertiary lymphoid structure 710 of FIG. 7A.

As the tertiary lymphoid structure 710 is closer to the tumor area 720 and the number thereof is greater, responsiveness or prognosis of the tumor area 720 to immune treatment may be more positive. Therefore, prediction accuracy of treatment responsiveness may be improved by generating the number of tertiary lymphoid structures 710 inside the tumor area 720 and the number of tertiary lymphoid structures 710 around the tumor area 720 as TLS information that is a quantified value.

In addition, for convenience of description, FIGS. 7A to 7C illustrate only one tertiary lymphoid structure 710, but one or more tertiary lymphoid structures 710 may be present in one tumor area 720 or stroma area 730, and accordingly, the processor may generate the number of tertiary lymphoid structures 710 around the tumor area 720 as TLS information.

FIGS. 8A to 8C are views illustrating classification according to maturation of a tertiary lymphoid structure, according to an embodiment of the present disclosure.

In an embodiment, a processor may generate the number of tertiary lymphoid structures forming a germinal center as TLS information. In detail, the processor may calculate the number of tertiary lymphoid structures forming the germinal center from maturation of a tertiary lymphoid structure that is a first parameter.

In an embodiment, the tertiary lymphoid structure may be classified into a lymphoid aggregate, a primary follicle, and a secondary follicle according to the maturation.

FIGS. 8A to 8C illustrate tertiary lymphoid structures in lymphoid follicle, primary follicle, and secondary follicle states, respectively.

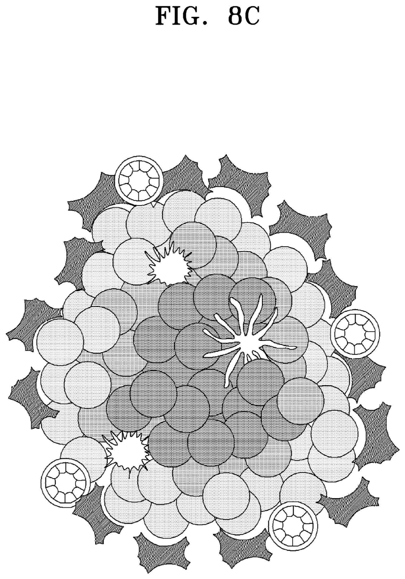

When B cells in a tertiary lymphoid structure receive a proliferation command from T cells, the B cells become in a primary follicle state and actively proliferate repeatedly. As a result of the repeated proliferation of the B cells, a germinal center is formed at the center of the tertiary lymphoid structure, and a portion to which a primary follicle is pushed out becomes a mantle zone. Referring to FIG. 8C, the germinal center is formed in the tertiary lymphoid structure in the secondary follicle state.

As tertiary lymphoid structures in the secondary follicle state are more present from among the tertiary lymphoid structures, responsiveness or prognosis of a tumor area to immune treatment may be more positive. Accordingly, a processor may generate, as TLS information, the number of tertiary lymphoid structures in the secondary follicle state, which form the germinal center, from among tertiary lymphoid structures identified from a pathological slide image. In addition, the processor may generate, as TLS information, a ratio of the number of tertiary lymphoid structures in the secondary follicle state to the number of tertiary lymphoid structures identified from the pathological slide image.

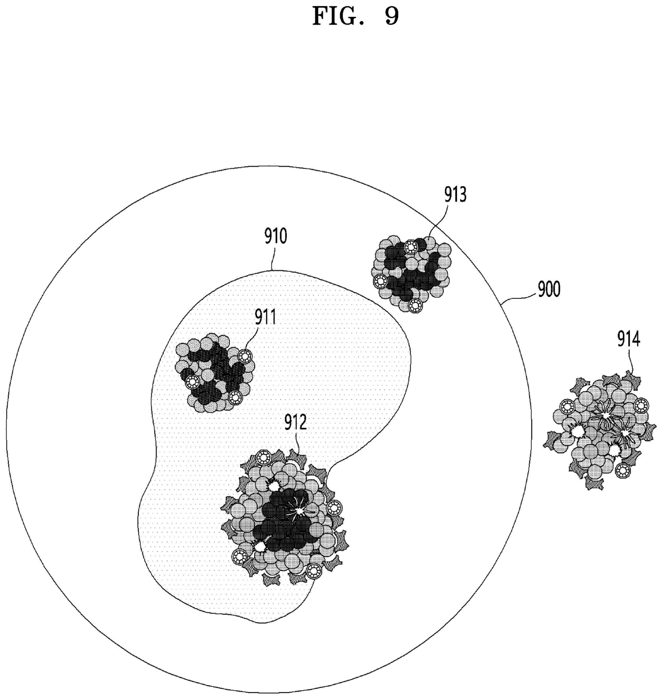

FIG. 9 is a view illustrating a region of interest according to an embodiment of the present disclosure.

FIG. 9 illustrates a region of interest 900, a tumor area 910, and a plurality of tertiary lymphoid structures 911 to 914.

In an embodiment, a processor may determine the region of interest (ROI) 900 on the basis of a distance from the tumor area 910. In the case where a purpose is to predict treatment responsiveness by analyzing a pathological slide image and detecting a pathologically significant biomarker, an area in which the tumor area 910 is not present may need to be excluded from an analysis target in terms of efficiency of analysis. Accordingly, as an example, the region of interest 900 may be preset, automatically set, or set on the basis of a user input, according to a type of tumor cell forming the tumor area 910, whether or not the tumor area 910 is present, a distribution of the tumor area 910, and the like. For example, the processor may determine, as the region of interest 900, an area at a distance within 2 mm from the tumor area 910.

In an embodiment, the processor may generate TLS information related to the tertiary lymphoid structures 911, 912, and 913 within the region of interest 900. In other words, the processor may generate only the TLS information related to the tertiary lymphoid structures 911, 912, and 913 within the region of interest 900, and may not generate TLS information related to the tertiary lymphoid structure 914 outside the region of interest 900.

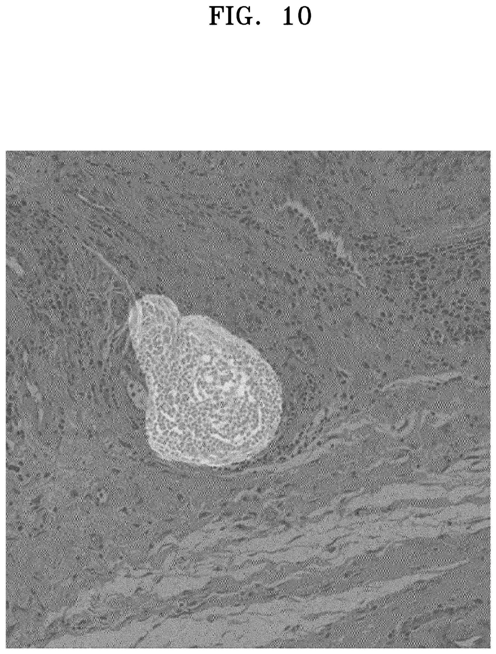

FIG. 10 is a view illustrating an example of an image displaying TLS information, according to an embodiment of the present disclosure.

In an embodiment, a processor may output, in a pathological slide image, an image including a visual image representing TLS information. As an example, the processor may output an image showing a detected tertiary lymphoid structure and TLS information corresponding to the detected tertiary lymphoid structure.

For example, the processor may output, in the pathological slide image, an image in which a region of interest is overlaid with a visual image representing TLS information. In other words, the processor may output, in the pathological slide image in which the visual image representing the TLS information is overlaid, the image so that one or more regions of interest are displayed on a display apparatus.

Referring back to FIG. 3, in operation 330, the processor predicts treatment responsiveness of a lesion associated with the pathological slide image, on the basis of TLS information.

In detail, the processor may classify a class of the pathological slide image on the basis of a preset cut-off value (cut-off value) for the TLS information. For example, the cut-off value may be determined, for each of the TLS information, as a value that improves prediction accuracy of treatment responsiveness.

In an embodiment, the processor may classify the pathological slide image into at least two classes on the basis of the number of tertiary lymphoid structures per unit area of the tumor area and a first cut-off value. In detail, the processor may compare the number of tertiary lymphoid structures per unit area of the tumor area with the first cut-off value, in the case where the number of tertiary lymphoid structures is greater than or equal to the first cut-off value, determine the class of the pathological slide image as "High," and in the case where the number of tertiary lymphoid structures is less than the first cut-off value, determine the class of the pathological slide image as "Low." The first cut-off value may be determined as a value that improves the prediction accuracy of the treatment responsiveness, on the basis of a clinical trial value for the number of tertiary lymphoid structures per unit area of the tumor area.

In an embodiment, the processor may classify the pathological slide image into at least two classes on the basis of a ratio of an area of the tumor area to an area of the tertiary lymphoid structure and a second cut-off value. In detail, the processor may compare the ratio of the area of the tumor area to the area of the tertiary lymphoid structure with the second cut-off value, and in the case where the ratio is greater than or equal to the second cut-off value, determine the class of the pathological slide image as "High," and in the case where the ratio is less than the second cut-off value, determine the class of the pathological slide image as "Low." The second cut-off value may be determined as a value that improves prediction accuracy of treatment responsiveness, on the basis of a clinical trial value for the number of tertiary lymphoid structures per unit area of the tumor area.

In an embodiment, the processor may classify the pathological slide image into at least two classes on the basis of the number of tertiary lymphoid structures inside/around the tumor area and a third cut-off value. As an example, the processor may classify the pathological slide image into three classes by comparing the number of tertiary lymphoid structures inside/around the tumor area with the third cut-off value and a fourth cut-off value. In detail, the processor may determine the class of the pathological slide images as "TLS structured" or "TLS included" indicating that the tertiary lymphoid structures are more present inside the tumor area than around the tumor area in the case where the number of tertiary lymphoid structures inside the tumor area is greater than or equal to the third cut-off value and the number of tertiary lymphoid structures around the tumor area is less than the fourth cut-off value, determine the class of the pathological slide image as "TLS excluded" indicating that the tertiary lymphoid structures are more present around the tumor area than inside the tumor area in the case where the number of tertiary lymphoid structures around the tumor area is greater than or equal to the fourth cut-off value and the number of tertiary lymphoid structures inside the tumor area is less than the third cut-off value, and determine the class of the pathological slide image as "TLS desertic" or "TLS desert" in the case where the number of tertiary lymphoid structures inside the tumor area is less than the third cut-off value and the number of tertiary lymphoid structures around the tumor area is less than the fourth cut-off value. The third cut-off value and the fourth cut-off value may be determined as values that improve the prediction accuracy of the treatment responsiveness, on the basis of clinical trial values for the number of tertiary lymphoid structures inside/around the tumor area.

In an embodiment, the processor may classify the pathological slide image into at least two classes on the basis of a distance between the tumor area and the tertiary lymphoid structure, a fifth cut-off value, and a sixth cut-off value. As an example, the processor may determine a type of the tertiary lymphoid structure on the basis of a distance between the tumor area and the tertiary lymphoid structure, and classify the pathological slide image into at least two classes on the basis of the type of the tertiary lymphoid structure. In detail, the processor may compare a mean of distances between the tumor area and the tertiary lymphoid structures (which may be, as another example, an intermediate value, a minimum distance, a maximum distance, or the like) with the fifth cut-off value, determine a type of a tertiary lymphoid structure, which is less than the fifth cut-off value, as "in," a type of a tertiary lymphoid structure, which is greater than or equal to the fifth cut-off value and less than the sixth cut-off value, as "near-out," and a type of a tertiary lymphoid structure, which is greater than or equal to the sixth cut-off value, as "far-out." In addition, the processor may classify the class of pathological slide image as "High" or "Low" on the basis of the types of the tertiary lymphoid structures and the number or a ratio of tertiary lymphoid structures corresponding to the respective types. Here, as an example, the mean of the distances between the tumor area and the tertiary lymphoid structures (which may be, as another example, an intermediate value, a minimum distance, a maximum distance, or the like) may be determined on the basis of a distance measured on the basis of a boundary of the tumor area. The fifth cut-off value and the sixth cut-off value may be determined as values that improve the prediction accuracy of the treatment responsiveness, on the basis of clinical trial values for the distances between the tumor area and the tertiary lymphoid structures.

In an embodiment, the processor may classify the pathological slide image into at least two classes on the basis of a distance between a plurality of tertiary lymphoid structures and a seventh cut-off value. In detail, the processor may compare a mean of distances among centers of the plurality of tertiary lymphoid structures (which may be, as another example, an intermediate value, a minimum distance, a maximum distance, or the like) with the seventh cut-off value, in the case where the mean is greater than or equal to the seventh cut-off value, determine the class of the pathological slide image as "Dispersed," and in the case where the mean is less than the seventh cut-off value, determine the class of the pathological slide image as "Crowded." The seventh cut-off value may be determined as a value that improves the prediction accuracy of the treatment responsiveness, on the basis of a clinical trial value for the number of tertiary lymphoid structures per unit area of the tumor area.

In an embodiment, the processor may classify the pathological slide image into at least two classes on the basis of a size of the tertiary lymphoid structure or the number of tertiary lymphoid structures forming a germinal center and an eighth cut-off value. In detail, the processor may compare the size of the tertiary lymphoid structure with the eighth cut-off value, in the case where the size is greater than or equal to the eighth cut-off value, determine the class of the pathological slide image as "Mature," and in the case where the size is less than the eighth cut-off value, determine the class of the pathological slide image. Alternatively, the processor may compare the number of tertiary lymphoid structures forming the germinal center with the eighth cut-off value, in the case where the number is greater than or equal to the eighth cut-off value, determine the class of the pathological slide image as "Mature," and in the case where the number is less than the eighth cut-off value, determine the class of the pathological slide image "Immature." The eighth cut-off value may be determined as a value that improves the prediction accuracy of the treatment responsiveness, on the basis of a clinical trial value for the size of the tertiary lymphoid structure or the number of tertiary lymphoid structures forming the germinal center.

In an embodiment, the processor may classify the pathological slide image into at least two classes on the basis of the number of tumor infiltrating lymphocytes located within a certain radius from the tertiary lymphoid structure and a ninth cut-off value. In detail, the processor may compare the number of tumor infiltrating lymphocytes located within the certain radius from the tertiary lymphoid structure with the ninth cut-off value, in the case where the number is greater than or equal to the ninth cut-off value, determine the class of the pathological slide image as "TIL rich," and in the case where the number is less than the ninth cut-off value, determine the class of the pathological slide image as "TIL poor." The ninth cut-off value may be determined as a value that improves the prediction accuracy of the treatment responsiveness, on the basis of a clinical trial value for the number of tumor infiltrating lymphocytes located within the certain radius from the tertiary lymphoid structure.

In an embodiment, the processor may classify the pathological slide image into at least two classes on the basis of a density of immune cells within the tertiary lymphoid structure and a tenth cut-off value. In detail, the processor may compare the density of the immune cells within the tertiary lymphoid structure with the tenth cut-off value, in the case where the density is greater than or equal to the tenth cut-off value, determine the class of pathological slide image as "Dense TLS," and in the case where the density is less than the tenth cut-off value, determine the class of the pathological slide image as "Sparse TLS." The tenth cut-off value may be determined as a value that improves the prediction accuracy of the treatment responsiveness, on the basis of a clinical trial value for the density of the immune cells within the tertiary lymphoid structure.

In an embodiment, the processor may output a class (or TLS information) of the pathological slide image and information associated with an immune phenotype together in the pathological slide image. The processor may determine at least one of the immune phenotype of the pathological slide image (or at least one region of interest within the pathological slide image) or the information associated with the immune phenotype. The information associated with the immune phenotype may include at least one of a plurality of classes (e.g., immune inflamed, immune excluded, immune desert) representing an immune environment of the pathological slide image, at least one of scores for the plurality of classes representing the immune environment (e.g., a probability that an immune phenotype of at least one region of interest corresponds to a corresponding class), or at least one of features representing the immune environment of the pathological slide image.

Hereinafter, an embodiment in which the processor predicts treatment responsiveness to an immune anticancer agent on the basis of a density of immune cells is described. Here, the processor may determine an immune phenotype for each of at least one area within the pathological slide image.

In detail, in the case where a density of immune cells within a tumor area is greater than or equal to a first threshold density, the processor may determine an immune phenotype of a corresponding area of the pathological slide image as immune inflamed. In addition, in the case where the density of the immune cells within the tumor area is less than the first threshold density and a density of immune cells within a stroma area is greater than or equal to a second threshold density, the processor may determine an immune phenotype of a corresponding area within the pathological slide image as immune excluded. In addition, in the case where the density of the immune cells within the tumor area is less than the first threshold density and the density of the immune cells within the stroma area is less than the second threshold density, the processor may determine an immune phenotype of a corresponding area within the pathological slide image as immune desert.

Accordingly, in the case where an immune phenotype the most included within the pathological slide image is immune inflamed, the processor may predict that a patient associated with the corresponding pathological slide image responds to an immune anticancer agent (i.e., the patient is a responder). In contrast, in the case where the immune phenotype the most included within the pathological slide image is immune excluded or immune desert, the processor may predict that the patient associated with the corresponding pathological slide image does not respond to the immune anticancer agent (i.e., the patient is a non-responder).

In an embodiment, the processor may determine a class of the pathological slide image on the basis of a preset cut-off value (e.g., the first to tenth cut-off values described above) for TLS information, and determine a class representing an immune phenotype of the pathological slide image. In addition, the processor may output the class of the pathological slide image and the class representing the immune phenotype of the pathological slide image. For example, the processor may determine the class of the pathological slide image as "Mature," determine the immune phenotype of the pathological slide image as immune inflamed, and then output the result of the determination of the pathological slide image as "Inflamed-Mature."

In an embodiment, the processor may determine TLS information by analyzing at least two pathological slide images stained with different reagents (e.g., hematoxylin and eosin (H&E) stain and immunohistochemistry (IHC) stain).

The method by which the processor classifies the class of the pathological slide image on the basis of the preset cut-off value (e.g., the first to tenth cut-off values described above) for the TLS information is not limited to the example described above.

As an example, the processor may classify a class corresponding to at least one region of interest by determining a class corresponding to each of at least one region of interest included in the pathological slide image on the basis of the preset cut-off value for the TLS information (e.g., the first to tenth cut-off values described above).

In an embodiment, the processor may assign a weight to the generated TLS information and classify the class of pathological slide image on the basis of the weight. For example, in the case where the processor assigns a greater weight to the number of tertiary lymphoid structures per unit area of a tumor area than to a ratio of an area of the tumor area to an area of a tertiary lymphoid structure, the processor may determine, as "High," the class of pathological slide image in which the number of tertiary lymphoid structures per unit area of the tumor area is greater than or equal to a first cut-off value, according to the assigned weight, even in the case where the ratio of the area of the tumor area to the area of the tertiary lymphoid structure is less than a second cut-off value. Meanwhile, the weight assigned to the TLS information may be determined according to clinical importance of each of the TLS information.

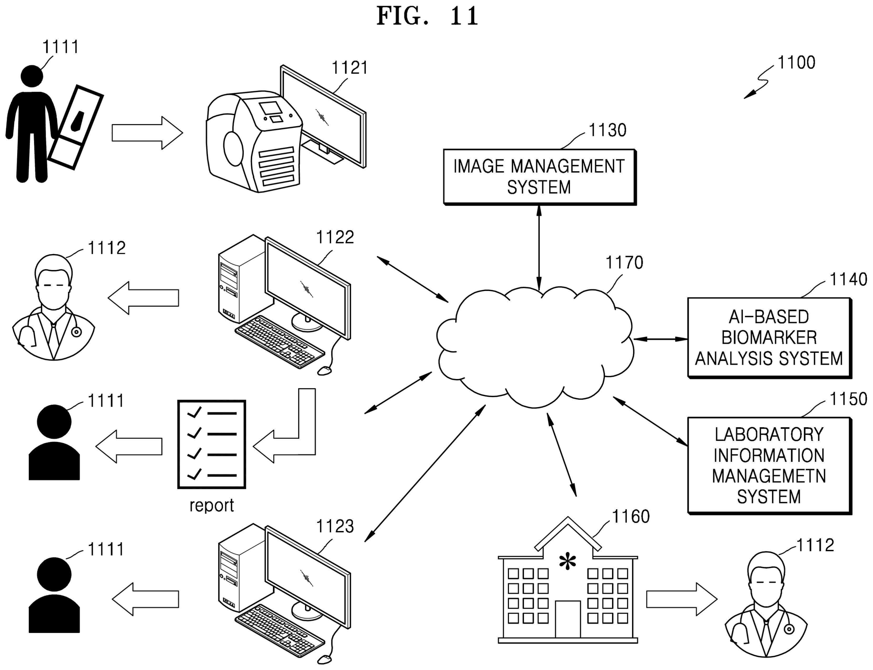

FIG. 11 is a view illustrating another example of a system for analyzing a pathological slide image.

Referring to FIG. 11, a system 1100 refers to an example of a system and a network for providing, processing, and reviewing slide images of tissue specimens by using an artificial intelligence model.

According to various embodiments of the present disclosure, the method described above with reference to FIGS. 2A to 10 may be performed by at least one or a combination of user terminals 1122 and 1123, an image management system 1130, an Al-based biomarker analysis system 1140, a laboratory information management system 1150, and a hospital or laboratory server 1160.

A scanner 1121 may acquire a digitized image from a tissue sample slide generated by using a tissue sample of a subject 1111. For example, each of the scanner 1121, the user terminals 1122 and 1123, the image management system 1130, the Al-based biomarker analysis system 1140, the laboratory information management system 1150, and/or the hospital or laboratory server 1160 may be connected to a network 1170, such as the Internet, via one or more computers, servers, and/or mobile devices or may communicate with a user 1112 via one or more computers, and/or mobile devices.

The user terminals 1122 and 1123, the image management system 1130, the Al-based biomarker analysis system 1140, the laboratory information management system 1150, and/or the hospital or laboratory server 1160 may generate tissue samples of one or more subjects 1111, tissue sample slides (pathological sides), digitized images of the tissue sample slides (the pathological slides), or any combination thereof, or otherwise, acquire the same from another apparatus. In addition, the user terminals 1122 and 1123, the image management system 1130, the Al-based biomarker analysis system 1140, the laboratory information management system 1150, and/or the hospital or laboratory server 1160 may acquire any combination of subject-specific information, such as age, medical history, cancer treatment history, family history, past biopsy records of the subject 1111, or disease information of the subject 1111.

The scanner 1121, the user terminals 1122 and 1123, the Al-based biomarker analysis system 1140, the laboratory information management system 1150, and/or the hospital or laboratory server 1160 may transmit a digitized pathological slide image, subject-specific information, and/or the result of analyzing the digitized pathological slide image to the image management system 1130 through the network 1170. The image management system 1130 may include a repository for storing a received image and a repository for storing a result of analysis.

In addition, according to various embodiments of the present disclosure, an artificial intelligence model, which is learned and trained to predict, from a pathological slide image of the subject 1111, at least one of information regarding at least one cell, information regarding at least one area, information related to a biomarker, medical diagnosis information, and/or medical treatment information, may be stored in the user terminals 1122 and 1123, the image management system 1130, and the like and operate.

As described above, a computer apparatus according to an embodiment of the present disclosure may generate information related to a tertiary lymphoid structure from a pathological slide image and automatically predict treatment responsiveness of a patient on the basis of the information related to the tertiary lymphoid structure. Therefore, an analysis method by which a pathologist directly examines a portion of a distribution of a tissue in a whole pathological slide image may be replaced. In addition, prediction accuracy and analysis speed of treatment responsiveness of a patient to an immune anticancer agent may be improved.

Meanwhile, the above-described method may be written as a program that may be executed on a computer, and may be implemented in a general-purpose digital computer that operates the program by using a computer-readable recording medium. In addition, a structure of data used in the above-described method may be recorded in a computer-readable recording medium via various types of means. The computer-readable recording medium includes a storage medium, such as a magnetic storage medium (e.g., ROM, RAM, a USB, a floppy disk, a hard disk, or the like) and an optical reading medium (e.g., CD-ROM, DVD, or the like).

Those skilled in the art related to the present embodiment may understand that the present embodiment may be implemented in a modified form within the scope that does not depart from the essential characteristics of the above description. Therefore, the disclosed methods should be considered in an illustrative rather than a restrictive sense, and the scope of the present disclosure should be defined by claims rather than by the foregoing description, and should be construed to include all differences within the scope equivalent thereto.

## Claims

1. A computing apparatus comprising:
at least one memory; and
at least one processor, wherein the at least one processor is configured to identify at least one tertiary lymphoid structure (TLS) from a pathological slide image, generate information related to the at least one tertiary lymphoid structure, and predict treatment responsiveness of a lesion associated with the pathological slide image, on the basis of the information.

2. The computing apparatus of claim 1, wherein the at least one processor is further configured to identify, from the pathological slide image, a component comprising at least one of a tumor area, a stroma area, and an immune cell.

3. The computing apparatus of claim 2, wherein the at least one processor is further configured to calculate a quantified value on the basis of at least one of a first parameter related to the tertiary lymphoid structure and a second parameter related to the component.

4. The computing apparatus of claim 3, wherein the first parameter comprises at least one of a number of the tertiary lymphoid structures, a location of the tertiary lymphoid structure, an area of the tertiary lymphoid structure, and maturation of the tertiary lymphoid structure, and the second parameter comprises at least one of a location of the tumor area, an area of the tumor area, a location of the stroma area, an area of the stroma area, a location of the immune cell, and a number of the immune cells.

5. The computing apparatus of claim 3, wherein the quantified value comprises a value reflecting structural information associated with the treatment responsiveness of the tertiary lymphoid structure within the pathological slide image.

6. The computing apparatus of claim 5, wherein the quantified value comprises at least one of a number of the tertiary lymphoid structures per unit area of the tumor area, a ratio of an area of the tumor area to an area of the tertiary lymphoid structure, a number of the tertiary lymphoid structures inside the tumor area, a number of the tertiary lymphoid structures around the tumor area, a distance between the tumor area and the tertiary lymphoid structure, a distance between a plurality of tertiary lymphoid structures, a size of the tertiary lymphoid structure, a number of the tertiary lymphoid structures forming a germinal center, a number of tumor infiltrating lymphocytes (TILs) located within a certain radius from the tertiary lymphoid structure, and a density of the immune cell within the tertiary lymphoid structure.

7. The computing apparatus of claim 1, wherein the processor is further configured to generate the information related to the tertiary lymphoid structure within a region of interest determined on the basis of a distance from the tumor area.

8. The computing apparatus of claim 1, wherein the processor is further configured to classify a class of the pathological slide image on the basis of a preset cut-off value for the information.

9. The computing apparatus of claim 1, wherein the processor is further configured to identify the at least one tertiary lymphoid structure by using an artificial intelligence model trained on the basis of a plurality of training images, and the plurality of training images are obtained by labeling the tertiary lymphoid structure on the pathological slide image.

10. A method of analyzing a pathological slide image, the method comprising:
identifying at least one tertiary lymphoid structure from a pathological slide image;
generating information related to the at least one tertiary lymphoid structure; and
predicting treatment responsiveness of a lesion associated with the pathological slide image, on the basis of the information.

11. The method of claim 10, further comprising identifying, from the pathological slide image, a component comprising at least one of a tumor area, a stroma area, and an immune cell.

12. The method of claim 11, wherein the generating comprises calculating a quantified value on the basis of at least one of a first parameter related to the tertiary lymphoid structure and a second parameter related to the component.

13. The method of claim 12, wherein the first parameter comprises at least one of a number of the tertiary lymphoid structures, a location of the tertiary lymphoid structure, an area of the tertiary lymphoid structure, and maturation of the tertiary lymphoid structure, and the second parameter comprises at least one of a location of the tumor area, an area of the tumor area, a location of the stroma area, an area of the stroma area, a location of the immune cell, and a number of the immune cells.

14. The method of claim 12, wherein the quantified value comprises a value reflecting structural information associated with the treatment responsiveness of the tertiary lymphoid structure within the pathological slide image.

15. The method of claim 14, wherein the quantified value comprises at least one of a number of the tertiary lymphoid structures per unit area of the tumor area, a ratio of an area of the tumor area to an area of the tertiary lymphoid structure, a number of the tertiary lymphoid structures inside the tumor area, a number of the tertiary lymphoid structures around the tumor area, a distance between the tumor area and the tertiary lymphoid structure, a distance between a plurality of tertiary lymphoid structures, a size of the tertiary lymphoid structure, a number of the tertiary lymphoid structures forming a germinal center, a number of tumor infiltrating lymphocytes located within a certain radius from the tertiary lymphoid structure, and a density of the immune cell within the tertiary lymphoid structure.

16. The method of claim 10, wherein the generating comprises generating the information related to the tertiary lymphoid structure within a region of interest determined on the basis of a distance from the tumor area.

17. The method of claim 10, wherein the predicting comprises classifying a class of the pathological slide image on the basis of a preset cut-off value for the information.

18. The method of claim 10, wherein the identifying comprises identifying the at least one tertiary lymphoid structure by using an artificial intelligence model trained on the basis of a plurality of training images, and the plurality of training images are obtained by labeling the tertiary lymphoid structure on the pathological slide image.

19. A computer-readable recording medium having recorded thereon a program for causing a computer to execute the method of claim 10.
